# EUROPEAN PATENT APPLICATION

(11) **EP 2 462 925 A1**
(43) Date of publication of application: **13.06.2012**
(21) Application number: 10191101.4
(22) Date of filing: 12.11.2010
(51) Int. Cl.: A61K 9/20, A61K 9/50

(54) **Pramipexole Dihydrochloride Granulate**

(71) Applicant: Neuraxpharm Arzneimittel GmbH, 40764 Langenfeld (DE)
(72) Inventor: Sewarte-Ross, Günter, 44145 Dortmunt (DE)
(74) Representative: Dörries, Hans Ulrich

(57) **Abstract**

A process for preparing a pramipexole dihydrochloride granulate comprising the steps of:
(a) applying a first solution, comprising pramipexole dihydrochloride or a pharmaceutically acceptable solvate thereof and a binding agent, onto particles of pre-blend ingredients to form a granulate of coated particles,
(b) drying the granulate of coated particles, and
(c) applying a second solution comprising a binder onto the dried granulate of coated particles to form the pramipexole dihydrochloride granulate.

## Description

### Field of Invention

The present invention relates to a process for preparing a pramipexole dihydrochloride granulate useful for the production of pramipexole tablets with pramipexole dihydrochloride monohydrate as active pharmaceutically ingredient (API). In particular, the present invention relates to a manufacturing process of a physically stable, suitably flowing granulate with suitable properties concerning the downstream compacting process. The granulate is used as a base granulate for all tablet strengths. Different amounts of base granulate are used for all tablets according to their strengths.

### Background of the Invention

Pramipexole is a known dopamine D2 receptor agonist and was originally disclosed in U.S. Pat. Nos. 4,731,374; 4,843,086 and 4,886,812. It has the molecular formula C₁₀H₁₇N₃S and a relative molecular mass of 211.33. The chemical formula of pramipexole is (S)-2-amino-4,5,6,7-tetrahydro-6-(propylamino)benzothiazole:

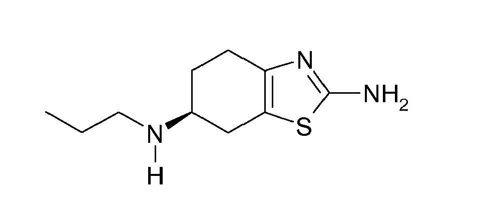

Pramipexole immediate release tablets were first authorized in the USA in 1997. They are indicated in the EU and US for the treatment of signs and symptoms of Parkinson's Disease. The product is known in the USA under the brand name MIRAPEX^{®}.

The production of pramipexole immediate release tablets typically involves the formation of a granulate, mixing the granulate with a suitable post-blend to a press-mass, and compressing the press-mass in a tablet press. There are a lot of manufacturing processes known for producing granulates to yield homogenous und physically stable tablets. But there are very few manufacturing processes known for producing granulates showing sufficient physical stability, sufficient flow properties and sufficient compacting properties, in particular in case of low-dosage granulates or low-dosage tablets. In this context a standard manufacturing process is commonly used, which involves fluid-bed granulation by spraying the dissolved API together with a suitable binding agent e.g. povidone solution onto the fluidized loading.

The granules resulting from the standard manufacturing process, in particular in the case of pramipexole dihydrochloride granulates, often suffer from unsuitable shape and size needed for further downstream processes. There was no amelioration achievable with any known technology. However, well shaped granules are in particular essential for sufficient flow properties, especially in the case of a fine granulate.

A process for preparing pramipexole dihydrochloride immediate release tablets with high storage stability in terms of chemical stability of the API is disclosed in WO 2008/023027 A2, published on Feb. 28, 2008. This process, however, does not disclose or make use of pramipexole dihydrochloride granules with suitable shape and size, and in particular suitable physical stability, flow properties and compacting properties, for further downstream processes.

### Summary of the Invention

For purposes of this disclosure and invention hereinafter the term pramipexole means pramipexole dihydrochloride and the pharmaceutically acceptable solvates thereof, in particular including the monohydrate of pramipexole dihydrochloride.

In accordance with the present invention, there is provided a manufacturing process for producing a pramipexole granulate wherein the granulate after drying shows improved physical stability, improved flow properties and improved compacting properties. The pramipexole granulate prepared according to the process of the invention yields homogenous und physically stable tablets after mixing with a suitable post-blend to a press-mass.

Within the present invention, there is further provided a pramipexole dihydrochloride granulate prepared according to the process of the invention.

Within the present invention, there is further provided the use of the pramipexole dihydrochloride granulate prepared according to the process of the invention to produce pramipexole dihydrochloride tablets.

Within the present invention, there is further provided a pramipexole dihydrochloride tablet prepared from the pramipexole dihydrochloride granulate according to the invention.

The process of the invention is described in independent claim 1, whereas beneficial modifications thereof are disclosed in the dependent claims. According to the invention, the process for preparing the pramipexole dihydrochloride granulate comprises the steps of:
(a) applying a first solution, comprising pramipexole dihydrochloride or a pharmaceutically acceptable solvate thereof and a binding agent, onto particles of pre-blend ingredients to form a granulate of coated particles,
(b) drying the granulate of coated particles, and
(c) applying a second solution comprising a binder onto the dried granulate of coated particles to form the pramipexole dihydrochloride granulate.

The pramipexole dihydrochloride granulate according to the invention has advantageous characteristics that particularly emerge after an optional drying step. It has surprisingly been found that while drying of the pramipexole dihydrochloride granulate according to the invention has an outstanding impact on the size of the granules, it does not negatively influence their shape. Dried pramipexole dihydrochloride granules according to the invention show improved properties in terms of smaller specific surface area, reduced porosity and more rounded, isometric shape. Moreover, the size of dried pramipexole dihydrochloride granules according to the invention approximately corresponds to the size of the coated particles (starting material). In other words, a downstream drying process surprisingly does not result in a buildup-granulation as expected, but in an amelioration of surface, porosity and shape, while the particle size is finally unchanged.

The consequences are better physical stability of the dried granules during downstream mechanical stress (e.g. conveying, blending, stress within the impeller dispenser of the tablet press), better flow properties resulting in reliable filling of a tablet press die (mass uniformity of tablets) and better compacting properties in terms of greater recovery of tablet crushing strength for a given compression force.

Preferably the process of the invention takes place in a fluid-bed granulator and involves fluid-bed granulation of the pre-blend ingredients with the API dissolved in water together with a suitable binding agent.

In a preferred embodiment, the pre-blend ingredients comprise starch, D-mannitol and colloidal silicon dioxide. The D-mannitol comprised in the pre-blend ingredients is preferably of EP or USP quality and preferably contains the beta crystal modification predominantly, more preferably contains not less than 50 % beta crystal modification and alpha crystals as remaining modification. Even more preferably D-mannitol contains solely beta crystal modification. The reason for this selection of crystal modifications is the superior stability of beta crystals followed by the extensive stability of the alpha crystals.

Suitable starches are rice, wheat, maize (corn), and potato starch, with maize starch being preferred. Other excipients could also be used, as will be readily apparent to a person skilled in the art.

The fraction of mass of D-mannitol within the loading is preferably more than 30%, more preferably between 50% and 70%. D-mannitol, preferably used as beta-modification, induces a unique precipitation of dissolved pramipexole within mannitol-maize starch blend. D-mannitol shows a prominent effect on pramipexol precipitation behaviour, which is why it can preferably be used in excess.

In a preferred embodiment, the starch of the pre-blend ingredients is pre-dried to a relative humidity of 4%. Maize starch, used in pre-dried quality, induces a unique precipitation of dissolved pramipexole within mannitol-maize starch blend. Due to minimizing formation of hydrate-pseudomodification of pramipexole, a water reduced quality of maize starch is essential.

In a preferred embodiment, the binding agent of the first solution is povidone. Povidone used in the spray solution as binding agent is also suitable to induce a unique precipitation of dissolved pramipexole within mannitol-maize starch blend.

In a preferred embodiment, the granulate of coated particles is skin-dried to a relative humidity of about 4 - 6 %. Skin-dried means a stage of drying, which is not complete. The inner volume of a granule contains some remaining humidity, whilst the outer surface is already dried completely. The term 'skin' refers to the outer surface of a granule. Preferably the coated particles should be dried to a large extent, to promote formation of a water free product. But drying below 4% rH could crumble down the granules within the granulator's turbulent air stream. Therefore the specified humidity range is a suitable compromise to generate the desired product.

In a preferred embodiment, the second solution comprises starch and water. Preferably, the present invention comprises the usage of maize starch within a maize starch paste with water with a suitable viscosity to spray onto a fluidized bed of prior processed particles as described above. The fraction of mass of maize starch within the granulate should preferably be less than 40%, more preferably between 3 % and 1 %. The maize starch paste comprised in the present invention is within a concentration range of 8% to 3% and preferably between 7% to 5%. The reason for this concentration range is the adjustment of a suitable viscosity for spraying the starch paste. Other starches such as rice, wheat, and potato starch could be used, as will be readily apparent to a person skilled in the art.

In a preferred embodiment, the resulting pramipexole dihydrochloride granulate is dried to a relative humidity of about 3 - 4 %. Preferably the granules should be dried to a large extent, to obtain a water free product. But drying below 3 % rH could promote disintegration of the granules within the granulator's turbulent air stream. Therefore the specified humidity range is a suitable compromise to generate the desired product.

The pramipexole dihydrochloride granulate of the present invention can be used to prepare pramipexole dihydrochloride capsules, tablets, in particular pramipexole dihydrochloride immediate release tablets, and the like. The pramipexole dihydrochloride and/or the pramipexole dihydrochloride granulate can be dosed in different amounts for the production of capsules and tablets of various strengths.

Preferably, the preparation of a pramipexole dihydrochloride tablet comprises the steps of mixing the pramipexole dihydrochloride granulate with post-blend ingredients to form a press-mass; and compressing the press-mass to tablets.

Preferably, the mixing step takes place in a free fall mixer. The post-blend ingredients can comprise mannitol, maize starch, colloidal silicon dioxide, povidone, and magnesium stearate. The resulting press-mass can be compressed to tablets using a tablet press.

### Brief Description of the Drawings

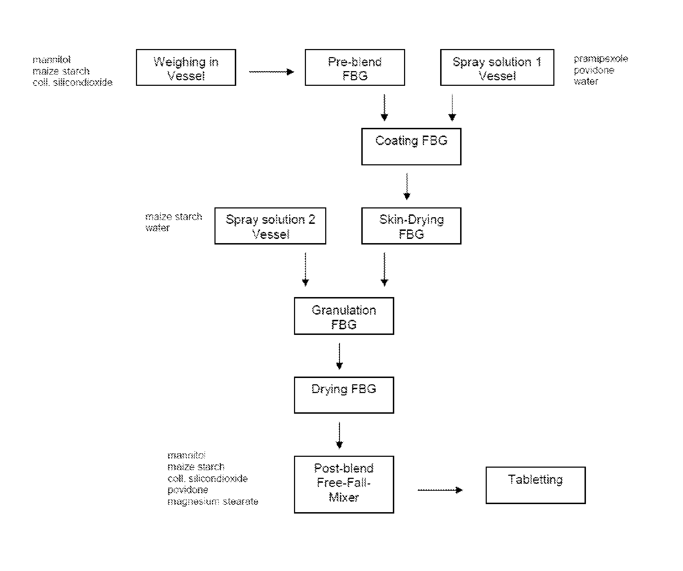

FIG. 1 shows a flow chart of a process for preparing a pramipexole dihydrochloride granulate according to a preferred aspect of the invention.

### Detailed Description of the Invention

In the flow chart of FIG. 1 a process for preparing a pramipexole dihydrochloride granulate according to a preferred aspect of the invention is shown. According to this process, pre-blend ingredients comprising mannitol, maize starch, and colloidal silicon dioxide are initially weighted into a fluid bed granulator (FBG). Subsequently, solution 1 comprising pramipexole, povidone as binding agent, and water is added, thereby coating the pre-blend ingredients to yield a granulate of coated particles. The granulate of coated particles is then dried, in particular skin-dried to a relative humidity of about 4 - 6 %.

After drying the granulate of coated particles, solution 2 comprising maize starch and water is added to the fluid bed granulator. The final pramipexole dihydrochloride granulate is then dried in the fluid bed granulator, in particular to a relative humidity of about 3 - 4 %.

The pramipexole dihydrochloride granulate according to the invention can be further processed to pramipexole dihydrochloride tablets, in particular pramipexole dihydrochloride immediate release tablets, by mixing it with post-blend ingredients, for example in a free fall mixer. The post-blend ingredients can comprise mannitol, maize starch, colloidal silicon dioxide, povidone, and magnesium stearate. The resulting press-mass can be compressed to tablets using a tablet press.

Table 1 shows a preferred composition of excipients of manufacturing levels.

**Table 1**

| **manufacturing level** | | | **item** |
|---|---|---|---|
| tablet | coated particles | loading FBG (pre-blend) | maize starch (predried to 4% rH) |
| | | | mannitol |
| | | | colloidal silicon dioxide |
| | | solution 1 (coating agent) | pramipexole |
| | | | povidone |
| | | | water (for povidone / API solution) |
| | granulate | solution 2 (binding agent) | maize starch, native |
| | | | water (for maize starch paste) |
| | post-blend | | mannitol |
| | | | maize starch (predried to 4% rH) |
| | | | povidone |
| | | | colloidal silicon dioxide |
| | | | magnesium stearate |

For example, pramipexole tablet strengths in the range of 0.125; 0.250; 0.500; 1.000; 1.500 mg can be produced this way. An overview of possible tablet strengths with compositions according to the invention is listed in table 2. Formulations are given as representative examples and are not limiting to this invention.

**Table 2, all readings in [mg/tablet]**

| | | **Tablet Strength** | | | | |
|---|---|---|---|---|---|---|
| **ID** | **item** | **0.125** | **0.250** | **0.500** | **1.000** | **1.500** |
| 1 | pramipexole | 0.125 | 0.250 | 0.500 | 1.000 | 1.500 |
| 2 | mannitol | 14.500 | 29.000 | 58.000 | 116.000 | 174.000 |
| 3 | maize starch (predried to 4% rH) | 7.750 | 15.500 | 31.000 | 62.000 | 93.000 |
| 4 | maize starch, native | 0.500 | 1.000 | 2.000 | 4.000 | 6.000 |
| 5 | colloidal silicon dioxide | 0.250 | 0.500 | 1.000 | 2.000 | 3.000 |
| 6 | povidone | 0.300 | 0.600 | 1.200 | 2.400 | 3.600 |
| 7 | water (for povidone / API solution) | 3.750 | 7.500 | 15.000 | 30.000 | 45.000 |
| 8 | water (for maize starch paste) | 6.750 | 13.500 | 27.000 | 54.000 | 81.000 |
| **9** | **granulate (dried)** | **23.425** | **46.850** | **93.700** | **187.400** | **281.100** |
| 10 | mannitol | 34.875 | 20.550 | 58.500 | 0.000 | 0.000 |
| 11 | maize starch (predried to 4% rH) | 24.250 | 15.700 | 43.000 | 10.000 | 15.000 |
| 12 | povidone | 0.750 | 0.450 | 1.200 | 0.000 | 0.000 |
| 13 | colloidal silicon dioxide | 0.600 | 0.350 | 1.000 | 0.000 | 0.000 |
| 14 | magnesium stearate | 1.100 | 1.100 | 2.600 | 2.600 | 3.900 |
| **15** | **press-mass** | **85.000** | **85.000** | **200.000** | **200.000** | **300.000** |

### Example

150 g pramipexole dihydrochloride monohydrate and 360 g povidone were dissolved in 4,500 g of purified water. 9,300 g maize starch, predried to 4 % rh, 17,400 g mannitol and 300 g colloidal silicon dioxide were sieved through a 2.4 mm sieve, placed in a fluid bed granulator and mixed. The pramipexole-povidone solution was sprayed onto the mixture in the fluid bed granulator and the resulting coated particles were skin dried to 4 % rh.

600 g maize starch was suspended in 8000 g water, heated to 80 °C and stirred until a solution was obtained. The solution was kept at a minimum temperature of 60 °C. It was sprayed onto the coated particles for granulation. The resulting granulate was dried to 3 %. The quantity of the resulting granules was 27,680 g corresponding to 98.5 % of the theoretical value.

It has been found that after the second granulation step the particle size of the granules had increased from about 100 µm up to 300 µm (d50). Measuring took place on granules dried on hurdles experimentally. The downstream drying process then had an outstanding impact on the properties of the granules in terms of size but not in terms of shape. The dried pramipexole dihydrochloride granules showed a reduced sized of about 100 µm. However, the dried pramipexole dihydrochloride granules showed improved properties in terms of smaller specific surface area, reduced porosity and more rounded, isometric shape, despite of the fact that the granules shrank or crumbled from 300µm to 100µm during the drying process. Moreover, the dried pramipexole dihydrochloride granules corresponded to the coated particles (starting material) in terms of their size (100µm).

The present invention is not to be limited in scope by the specific embodiments described herein, which are intended as single illustrations of individual aspects of the invention, and functionally equivalent methods and components are within the scope of the invention. Various modifications of the invention, in addition to those shown and described herein, will become apparent to those skilled in the art from the foregoing description and accompanying drawings. Such modifications are intended to fall within the scope of the appended claims.

## Claims

1. A process for preparing a pramipexole dihydrochloride granulate comprising the steps of:
(a) applying a first solution, comprising pramipexole dihydrochloride or a pharmaceutically acceptable solvate thereof and a binding agent, onto particles of pre-blend ingredients to form a granulate of coated particles,
(b) drying the granulate of coated particles, and
(c) applying a second solution comprising a binder onto the dried granulate of coated particles to form the pramipexole dihydrochloride granulate.

2. The process according to claim 1, wherein the pre-blend ingredients comprise starch, D-mannitol and colloidal silicon dioxide.

3. The process according to claim 2, wherein the D-mannitol of the pre-blend ingredients contains the beta crystal modification predominantly, preferably contains not less than 50 % beta crystal modification, and even more preferably contains solely beta crystal modification.

4. The process according to claim 3, wherein the D-mannitol of the pre-blend ingredients contains the alpha modification as the remaining modification.

5. The process according to any one of claims 2 to 4, wherein the fraction mass of the D-mannitol within the pre-blend ingredients is more than 30%, preferably between 50% and 70%.

6. The process according to any one of claims 2 to 5, wherein the starch of the pre-blend ingredients is pre-dried to a relative humidity of 4%.

7. The process according to any one of the preceding claims, wherein the binding agent of the first solution is povidone.

8. The process according to any one of the preceding claims, wherein the drying of the granulate of coated particles involves skin-drying to a relative humidity of about 4 - 6 %.

9. The process according to any one of the preceding claims, wherein the second solution comprises starch and water

10. The process according to claim 9, wherein the starch comprised in the second solution is within a concentration range of 8% to 3%, preferably of 7% to 5%, and/or wherein the fraction of mass of starch within the pramipexole dihydrochloride granulate is less than 40%, more preferably between 3 % and 1 %.

11. The process according to any one of the preceding claims, wherein the pramipexole dihydrochloride granulate is dried, preferably to a relative humidity of about 3 - 4 % and/or the particle size of the granulate of coated particles.

12. Pramipexole dihydrochloride granulate obtainable according to the process of any one of claims 1 to 11.

13. Use of the pramipexole dihydrochloride granulate of claim 12 to prepare pramipexole dihydrochloride tablets.

14. A process for preparing a pramipexole dihydrochloride tablet, comprising:
(i) preparing a pramipexole dihydrochloride granulate according to the process of any one of claims 1 to 11,
(ii) mixing the pramipexole dihydrochloride granulate with post-blend ingredients to form a press-mass, and
(iii) compressing the press-mass to tablets.

15. Pramipexole dihydrochloride tablet obtainable from the pramipexole dihydrochloride granulate of claim 12.
